# EUROPEAN PATENT APPLICATION

(11) **EP 1 980 255 A1**
(43) Date of publication of application: **15.10.2008**
(21) Application number: 06783808.6
(22) Date of filing: 27.07.2006
(51) Int. Cl.: A61K 31/57, A61K 31/593, A61K 33/06, A61P 19/10

(54) **PHARMACEUTICAL COMPOSITIONS CONTAINING SYNTHETIC STEROID DERIVATIVES SUCH AS TIBOLONE, MINERALS SUCH AS CALCIUM AND THE ACTIVE METABOLITE OF VITAMIN D (CALCITROL) FOR THE PREVENTION AND TREATMENT OF OSTEOPOROSIS AND MENOPAUSE**

(30) Priority: 20.12.2005 MX PA05014091
(71) Applicant: ESPINOZA ABDALA, Leopoldo de Jesús, Jalisco Mexico (MX); WORLD-TRADE IMPORT-EXPORT, WTIE, A.G., 6302 Zug (CH)
(72) Inventor: GARCÍA ARMENTA, María Elena, C.P. 45020 Guadalajara, Jalisco (MX); ALVAREZ OCHOA, Victor Guillermo, C.P. 45222 Zapopan, Jalisco, México (MX); SANTOS MURILLO, Josefina, C.P. 44600 Zapopan, Jalisco, México (MX); GARCIA ARMENTA, Patricia, C.P. 45110 Zapopan, Jalisco, México (MX)
(74) Representative: Carvajal y Urquijo, Isabel
(86) International application number: PCT/MX2006/000079
(87) International publication number: WO 2007/073135

(57) **Abstract**

The present invention generally relates to pharmaceutical industry and, in particular, to drug preparer pharmaceutical industry for controlling osteoporosis. More specifically, the present invention relates to a combination comprising a combination of a synthetic steroid derivative such as Tibolone, minerals such as calcium and the active metabolite of vitamin D 1,25(OH)₂ (calcitrol) for the prevention and treatment of osteoporosis. The inventive composition is advantageous over the prior art in that the same results can be obtained with a smaller amount of each of the components than when they are consumed separately or better results can be obtained with the same amount of each of the components than when they are consumed separately. Said composition consists of: a synergistic combination of a synthetic steroid, minerals and the active metabolite of vitamin D, 1,25 (OH)2 D3 (calcitrol) for the prevention and treatment of osteoporosis.

## Description

### FIELD OF THE INVENTION

The present invention generally relates to pharmaceutical industry and, in particular, to drug preparer pharmaceutical industry for controlling osteoporosis. More specifically, the present invention relates to a combination comprising a combination of a synthetic steroid derivative such as Tibolone, minerals such as calcium and the active metabolite of vitamin D 1,25(OH)₂ (calcitriol) for the prevention and treatment of osteoporosis.

### BACKGROUND OF THE INVENTION

Although not forming part of the official mortality statistics, osteoporosis is the second major public health problem considering the number of affected people and the economic costs of the disease treatment, only 27 percent of women aged 50 and older have a normal bone mass, 57 percent manifest osteopenia and 10 percent are estimated to already have osteoporosis.

Osteoporosis is defined as a progressive disease of the skeleton characterized by the loss of bone mass and microarchitectural deterioration and increased bone fragility, and consequent fracture risk.

The fracture risk is twice higher with a decreased density standard deviation. The risks is more than twice higher in low bone mass individuals, and four times higher in women with osteoporosis as for normal bone density women.

Patients who have suffered one ore more fractures at any site of the body are likely to develop subsequent fractures, regardless the bone density level. The earlier the age at which the first fractured was produced and the higher the number of previous fractures is, the higher the subsequent risk will be. Vertebral fractures risk increases twice in the presence of a history of these fractures. A hip fracture risk is twice higher with a wrist fracture history and triplicates the risk of developing vertebral fracture. A wide series of other clinical factors associated with a higher risk has been identified. Among them, genetic or constitution factors, nutritional factors and lifestyle, certain diseases (neurological and endocrine diseases) and drugs are mentioned.

### Types of Osteoporosis

**Hormonal Osteoporosis.** In some patients with osteoporosis, the underlying cause includes a hormonal imbalance produced by an increase of the antianabolic hormone secretion. Therefore, osteoporosis is characterized by hyperparatiroidism, hyperpituitarism, hypertiroidism, and hyperadrenocorticism (due to a hyperactivity of the suprarenal cortex, or as a result of an extended treatment with cortisol.

**Osteoporosis due to inactivity.** Any type of body becomes atrophied when it is not used, and bones are not an exception (excepting cranial bones); and can strike at any age. The intermittent pressures from the body support and the tensions from the muscle impulse transmitted to the skeleton exert forces and tensions which seem to stimulate the bone deposition through osteoblastic activity. In a person who, by whatever reason, is confined to bed or who has very limited activities, bone deposition is soon overcome by the resorption thereof, resulting thus in an atrophy due to bone inactivity (osteoporosis due to inactivity).

This type of osteoporosis, of course, is more marked at those sites of the skeleton which are less used, namely, the lower limbs and vertebral column. There is no doubt that the extended immobility of a limb, the lack of weight support and paralysis can result in a osteoporosis due to *localized* inactivity, limited to the bones which are not used.

**Postmenopausal and senile osteoporosis.** These two types of generalized osteoporosis are jointly studied as they have many common aspects. Their distinction is somewhat arbitrary, in a sense that when women develop an osteoporosis between menopause and aged 65, osteoporosis is called *postmenopausal,* while when men and women develop said process at the age of 65 years old, then it is called *senile.*

By far, postmenopausal and senile osteoporosis represents the generalized bone disease which can more frequently be observed among the patients. Osteoporosis has been calculated to be radiographically detectable in a 50% of people aged 65 or older and, when bearing in mind that the total bone amount should be reduced in a third before the decrease can be easily radiographically detected, it can be noted that the least severe levels of postmenopausal and senile osteoporosis are indeed quite frequent.

In the elderly, hypogonadism, as well as the inadequate calcium intake, seem to be the etiological factors of this type of osteoporosis and, moreover, the problem can become worse due to a further "osteoporosis due to inactivity" associated with the usual decreased clinical activity of the elderly.

**Sudek's Atrophy.** This is a localized osteoporosis, posttraumatic or subsequent to the immobility, accompanied by pain and sometimes by edema in the affected area and hypercalciuria. The lesion is reversible and the influence of a negative nervous factor (sympathetic) is evident. It is more frequently observed in bones of the hand and leg skeleton after fractures, severe distortion or extended immobility. It is never presented in children. Pathogenesis: Acidosis-anoxia-vasodilatation-stasis. This explains the presence of ruddiness and increased local sweat. It is appreciated in the radiography one type of osteoporosis in islets, with some normal density areas among them (osteoporosis moteadu).

### Most common fractures in Osteoporosis

The most common fractures are located in the wrist, column and, in a lesser extent, in the shoulder. There are three typical sites affected by osteoporosis: vertebral column, femoral neck and lower section of the forearm.

**Vertebral column fractures.** The causes for the appearance of these fractures are usually sudden and intense contractions of the muscles of the back. The appearance the vertebrae assume is a wedge due to infraction of the upper and lower angles from the vertebral body, preferably dorsal vertebrae. A more advance step is constituted by the flattening of the vertebral body which intensifies the existing dorsal kyphosis and reduces the patient's size. This type of fractures, fundamental to the reduction of height, can be presented in one of the discs, preferably the lower disc or collectively in both.

**Wrist Fractures.** The last group of fractures related to osteoporosis is that formed by ulna and radius Colle's fractures. Nordin, in (1973) have already commented the huge increase of the occurrences of these fractures in people aged 50 or older, especially in women, so he suggested a connection between the occurrences thereof and the bone losses in that life stage.

**Femoral Neck Fractures.** To many authors, the behavior of this area is identical to that from the vertebral column due to fact that the structure thereof is, for the most part, trabecular bone tissue. There are currently methods for determining bone mineral density, such as dual photon absorptiometry, dual x-ray absorptiometry, (with an accuracy error from 0.5 to 2%), quantitative computed tomography (which expresses density in grams per cm³), and the most modern which are called Neutron-Activation Analysis and Ultrasound.

The osteoporosis has severe repercussions on women's health and is one of the major causes of female morbimortality at present time, so the PREVENTION, early bone changes IDENTIFICATION, and timely APPLICATION of the therapeutic measures are fundamental aspects in the daily medical practice.

### TIBOLONE

Tibolone is a synthetic steroid with progestational, androgenic and estrogenic properties. Tibolone has showed to relieve climacteric symptoms, prevent osteoporosis appearance, without an impact on the endometrium and the volume of asymptomatic uterine myomas; it has also showed to induce a significant decrease in cholesterol and triglyceride serum levels.

After oral administration, Tibolone is rapidly metabolized into three compounds, which contribute to the pharmacodynamic profile. Tibolone has estrogenic, progestagenic and androgenic activities. Two of the metabolites (3a-OH-tibolone and 3β-OH tibolone) have a estrogenic activity, while the third metabolite (tibolone isomer D4) has progestagenic and androgenic activities. Due to tibolone combined activities and the metabolites thereof, various specific tissue effects are exerted: vasomotor effects, favorable effects over symptoms related to vaginal atrophy, increases bone mineral density, endometrium, breast and cardiovascular system.

After oral administration, tibolone is rapidly and completely absopted. Due to its fast metabolism, tibolone plasma levels are very low. Likewise, isomer D-4 plasma levels of tibolone are very low as well. The maximum plasma levels of metabolites 3a-OH y 3β-OH are higher and the achievement thereof is after 1 to 1.5 hours, the elimination semi-life thereof is from about 7 hours and accumulation is not observed. Tibolone is excreted mainly in the form of conjugated metabolites (mostly sulphated). Part of the administered compound is excreted through urine, but it is mostly expelled through feces. Food intake does not have significant effects over the absorption level.

It was found that the pharmacokinetic parameters for tibolone and the metabolites thereof are independent from the renal function.

### CALCIUM

Calcium is the most abundant mineral within the body. The main function of this mineral is bone and teeth construction. There is about 1 kg and 1.5 kg of calcium within the human body.

Calcium absorption by way of the mouth is about 30%, the active transport mechanisms act on the upper part of the small intestine. Calcium carbonate absorption is pH-dependant, gastric acid transforms calcium carbonate into calcium chloride which can be combined at the duodenum and jejunum, with the phosphorus ions ingested though food. The resulting insoluble phosphate is expelled through feces. Orally administered, calcium carbonate increases the passive calcium absorption and decreases that from phosphorus, being combined with the latter: The combination effect with phosphorus is pursued particularly in patients with renal insufficiency as the rest of the products which are combined with phosphorus (derived from aluminum or magnesium) expose the patients to certain toxicity.

As far as blood concerns, calcium administration causes an increase of calcemia and a decrease of phosphatemia. The decrease to this latter which also decreases phosphorus intracellular retention, has also as a consequence, as far as renal tubule concerns, a vitamin D activation, which propitiates an increase of the circulating calcitrol (vitamin D) concentrations compared to that which can be obtained with the control of hyperphosphatemia with aluminum derivatives.

Calcium is absorbed in the intestine in its soluble ionized form, which comprises two stages; first, calcium retention at the mucous pole and, second, calcium exit at the serous pole from the intestinal epithelium. Calcium retention by the mucous membrane is regulated by a carrier and by a protein which binds the calcium.

Calcium absorption is stimulated by vitamin D and by parathyroid hormone. Vitamin D is metabolized in the body, resulting in 1.25 dihydrocalciferol which is necessary for calcium active transport within the intestine; calcium excretion is performed by the kidney. Parathyroid hormone stimulates calcium reabsorption at renal level. Endogenous calcium and that ingested in foods or as a calcium supplement are excreted through feces.

Calcium absorption decreases with age and with estrogenic depletion during menopause. It increases from 10 to 30% when is taken with foods. The non-absorbed calcium is expelled through feces, saliva, bile, pancreatic juice and intestinal glands secretions, though urine and sweat. During lactation, amounts up to 34 mg of calcium/100 ml of milk are excreted in milk.

Vitamin D is absorbed within the intestine to be further transformed within the liver to 25(OH)D by microsomal enzymes. Then, it passes to the kidney, wherein 1-alpha-hydroxylation is performed, producing 1-alpha-dihydroxy-D. 1-alpha-25-dihydroxy-D has a regulating function on calcium and phosphorus metabolism, acting directly on intestinal receptors in order to increase calcium absorption. Due to the mentioned above, vitamin D has a direct and indirect action on cells taking part in the bone restructuring process.

### VITAMIN D₃

Vitamin D is essential for promoting calcium and phosphorus absorption and utilization for normal bone calcification. Along with parathyroid hormone and calcitocin, vitamin D regulates phosphate and serum calcium concentration as much as necessary. Vitamin D stimulates calcium and phosphate absorption from the small intestine and mobilizes calcium from bone.

Colecalciferol is transferred to the liver where it is converted to calciferiol (25-hydroxycolecalciferol), which is then transferred to the kidneys and converted to calcitriol (1,25-dihydroxycolecalciferol, thought to be the most active form) and 24,25-dihydroxycolecalciferol (physiologic role not determined).

Vitamin D is absorbed along with fats from the small intestine (in the jejunum and ileus) with the aid of the bile. Vitamin D formed in the skin by irradiation of the present provitamin, is directly absorbed into the bloodstream.

Vitamin D is excreted mainly in the bile from the small intestine, therefrom in the feces. Less than 4% of the intake is excreted in urine.

### OBJECTS OF THE INVENTION

One of the objects of the invention is to achieve a composition for treating osteoporosis, which allows, with the same results, a fewer amount of the components than when they are administered separately.

Another of the objects of the invention is to achieve a composition for treating osteoporosis, which allows, with increased results, a fewer amount of the components than when they are administered separately.

Other objects and advantages of the present invention would be apparent from the study of the following description and accompanying drawings with illustrative purposes only rather than limitative.

### BRIEF DESCRIPTION OF THE INVENTION

The present invention relates to the association or combination of a synthetic steroid derivative such as Tibolone, minerals such as calcium and the active metabolite of vitamin D 1,25(OH)₂ (calcitrol) for the prevention and treatment of osteoporosis.

The combination thereof produces a synergistic effect when they are applied in combination than when they applied independently, and there is a fewer dosage of each of these components, preventing accordingly side effects which can be presented when they are independently administered.

### DETAILED DESCRIPTION OF THE INVENTION

In accordance with the present invention, the pharmaceutical composition comprising a combination of synthetic steroids such as Tibolone, minerals such as calcium and the active metabolite of vitamin D, 1,25 (OH)₂ D₃, whose combination produces a synergistic effect, better tolerated, with fewer adverse effects in treatment and prevention of Osteoporosis.

### EXAMPLE 1

A closed, prospective, experimental, double-blind and crossover study was made in order to determine the effectiveness and reliability of Tibolone, calcium and vitamin D₃ in postmenopausal and oophorectomized women, to suppress climacteric symptoms without stimulating endometrial development and to assess the state of osteoporosis thereof and the changes in bone density, wherein 29 women aged 25-65 with natural or surgical menopause were included, who were daily administered the combination Tibolone/calcium/vitamin D₃ for 4 consecutive months and a placebo for four months for comparing the clinical answer between them; during the 8-month therapy, clinical parameters, sultriness scale, menopause index, Greene's scale, and bone density were registered.

### RESULTS

Both tibolone/calcium/vitamin D₃ and placebo produced a rapid and significant decrease of climacteric symptoms, even though the first one was greater than the placebo as to vasomotor symptoms and sexual activity. A significant increase in bone mineral density with the administration of the combination tibolone/calcium/ vitamin D₃ was showed. The changed to placebo produced a rapid loss of bone mineral density. An non-serious adverse event was reported characterized by uterine bleeding, which did not merit the suspension of the therapy under study.

### CONCLUSIONS

The combination tibolone/calcium/vitamin D₃ has shown to be effective in preventing osteoporosis, in addition to the association with calcium and vitamin D₃, being showed as an innovative drug considered as an alternative to the replacing hormonal therapy.

### EXAMPLE 2

An open, prospective study was made in order to determine the effects of tibolone/calcium/vitamin D₃ on the glucose tolerance curve and insulin secretion. 10 healthy postmenopausal patients who received the combination orally during tree months were included.

At the beginning and at the end of the study, glucose tolerance curves, insulin quantification at fasting and an hour after an oral 100 g glucose load and lipid profile (total cholesterol, triglycerides, HDL, LDL, Apo-LpA, Apo-LpB) were measured.

### RESULTS

The results showed a significant decrease in the levels of insulin at fasting, cholesterol and triglycerides three weeks after the treatment with tibolone/calcium/vitamin D₃. Glucose tolerance curve was not altered.

### CONCLUSIONS

The study showed that the combination Tibolone/calcium/vitamin D₃ reduces the presence metabolic markers of cardiovascular risk present in menopausal women.

### NOVELTY OF THE INVENTION

Having thus described the present invention, it is considered as a novelty and, therefore, it is related as property that contained in the following claims:

## Claims

1. Synergistic pharmaceutical composition comprising a synergistic combination of a synthetic steroid, minerals and the active metabolite of vitamin D, 1,25(OH)₂ D₃ (calcitrol) for preventing and treating Osteoporosis.

2. Pharmaceutical composition according to claim 1, comprising the combination of a synthetic steroid known as Tibolone, minerals known as calcium, and the active metabolite of vitamin D₃ known as 1, 25(OH)₂ D₃ (calcitrol).

3. A pharmaceutical composition according to claim 4, wherein calcium is in the form of calcium carbonate.

4. A pharmaceutical composition according to claim 1, 2, and 3, wherein vitamin D₃ is in the form of colecalciferol.

5. A pharmaceutical composition according to claim 1, wherein tibolone is present in a concentration of 2.5 mg per dosage unit.

6. A pharmaceutical composition according to claim 6, wherein calcium carbonate is present in a concentration of 500 mg per dosage unit.

7. A pharmaceutical composition according to claim 5, wherein calcitrol is present in a concentration of 400 UI per dosage unit.

8. Pharmaceutical compositions according to all previous claims, **characterized by** biphosphonate substances, etidronate, alendronate, risedronate, tiludronate, clodronate, pamidronate IV, fluorite, sodium fluoride, monofluorophosphate, selective strogen receptor modulators, raloxifene, benzothiophene derivatives are excluded from the formulation.

9. The use of the composition according to claim 2, for prevention and treatment of osteoporosis and control of menopausal symptoms.
